# EUROPEAN PATENT APPLICATION

(11) **EP 1 167 969 A2**
(43) Date of publication of application: **02.01.2002**
(21) Application number: 01304648.7
(22) Date of filing: 25.05.2001
(51) Int. Cl.: G01N 33/48, G06F 19/00

(54) **Method and system for predicting pharmacokinetic properties**

(30) Priority: 14.06.2000 US 211864 P
(71) Applicant: PFIZER INC., New York, N.Y. 10017 (US)
(72) Inventor: Hattori, Kazunari, Aichi-ken 470-2393 (JP); Shimada, Kaore, Aichi-ken 470-2393 (JP); Uchiyama, Mamoru, Aichi-ken 470-2393 (JP)
(74) Representative: Hayles, James Richard

(57) **Abstract**

This invention provides a method for predicting pharmacokinetic properties of molecules comprising the steps of:
(a) preparing 2D-structures of molecules used as a training set;
(b) constructing a 2D-fingerprint by counting the number of structural descriptors that potentially relate to a pharmacokinetic property, either manually or automatically using internally developed macro; wherein said structural descriptors consist of predefined 20 to 80 atoms/fragments or substructures;
(c) analyzing the obtained 2D-fingerprint by a statistical analysis method to correlate with the pharmacokinetic property of the molecule to yield a quantitative structure-property relationship (QSPR) model; and
(d) calculating the pharmacokinetic property of a trial molecule using the above obtained QSPR model.

A system for this invention is also provided. According to this method and system, it is possible to predict pharmacokinetic properties of molecules prior to synthesis, without labor-intensive and time-consuming experimentation.

## Description

### Technical Field

This invention relates to a method and system to predict pharmacokinetic (ADME) properties such as drug absorption (permeability), distribution, metabolism, and excretion, which are crucial properties in drug discovery.

### Background Art

Experimental measurements to obtain pharmacokinetic properties are time-consuming and labor-intensive. Moreover experiments require a significant amount of actual compounds. Thus, the computational methods to predict such properties of virtual compounds are highly desirable in prioritization of targets prior to synthesis.

So far, similar descriptors as conventionally employed in the quantitative structure activity relationship (QSAR) analysis (steric bulk, lipophilicity, HOMO energy, *etc.)* have been adopted in quantitative structure property relationship (QSPR) analysis to correlate with PK-related parameters (t1/2, clearance, or oxidation rate, *etc.)* (Lien, E. J. *et al. Acta Pharm. Jugosl.* **1984,** *34,* 123―131; Baeaernhielm, C. *et al. Chem.-Biol. Interact.* **1986,** 58, 277―288). Graph theory derived parameters (molecular connectivity indexes, *etc.)* have been also used for this purpose (Markin, R. *S. et al. Pharm. Res.* **1988,** *5,* 201―208; Garcia-March, F. J. *et al. J. Pharm. Pharmacol.* **1995,** *47,* 232―236). Recently reported QSPR methods necessitate calculation on 3D-structures that is still computationally intensive (Lombardo, F. *et al. J*. *Med. Chem.* **1996,** *39,* 4750-4755; Palm, K. *et al. J. Med. Chem.* **1998,** 41, 5382-5392; Clark, D. E. *J. Pharm. Sci.* **1999**, *88,* 815―821). The QSPR methods also necessitate complete set of molecular parameters (van de Waterbeemd, H. *et al. Quant. Struct.-Act. Relat.* **1996,** *15,* 480―490) that require experimental measurements to be determined.

2D-fingerprints are frequently employed in molecular similarity/diversity analysis *(e.g.* ISIS™/Base similarity search or SYBYL™/Selector), high-volume QSAR analysis *(e.g.* HQSAR, *vide infra),* and other drug discovery scenes. To date there has been no report on development of 2D-fingerprints descriptors to analyze pharmacokinetic properties.

WO 98/07107 discloses a MOLECULAR HOLOGRAM QSAR (HQSAR™) to develop high volume QSAR models. HQSAR™ uses molecular hologram based on fragments counts to deal with mostly potency/activity. A symposium proceeding (Niwa, T. "Prediction of Human Intestinal Absorption of Drug Based on Neural Network Modeling"; 27^{th} Symposium on Structure-Activity Relationships held in Japan, Nov. 10, 1999) describes a method to estimate human intestinal absorption (HIA) based on molecular topological indexes derived from 2D-structure.

It could be highly desirable to provide a system and method to predict pharmacokinetic properties of actual and virtual molecules with high performance (predictivity and speed) and wide applicability to diverse molecules.

### Brief Disclosure of the Invention

This invention provides a new method and system for QSPR analysis and prediction based on only 2D-structure that allows us to predict hundreds of compounds rapidly. The method and system of this invention employs 2D-fingerprints, an array of the counts of functional groups as descriptors for QSPR.

This invention provides a method for predicting pharmacokinetic properties of molecules comprising the steps of:
(a) preparing 2D-structures of molecules used as a training set;
(b) constructing a 2D-fingerprint by counting the number of structural descriptors that potentially relate to a pharmacokinetic property, either manually or automatically using internally developed macro; wherein said structural descriptors consist of predefined 20 to 80 atoms/fragments or substructures;
(c) analyzing the obtained 2D-fingerprint by a statistical analysis method to correlate with the pharmacokinetic property of the molecule to yield a quantitative structure-property relationship (QSPR) model; and
(d) calculating the pharmacokinetic property of a trial molecule using the above obtained QSPR model.

This invention also provides a system for predicting pharmacokinetic properties of molecules comprising:
(a) means for preparing 2D-structures of molecules used as a training set;
(b) means for constructing a 2D-fingerprint by counting the number of structural descriptors that potentially relate to a pharmacokinetic property, wherein said structural descriptors consist of predefined 20 to 80 atoms/fragments or substructures;
(c) means for analyzing the obtained 2D-fingerprint by a statistical analysis method to correlate with the pharmacokinetic property of the molecule to yield a quantitative structure-property relationship (QSPR) model; and
(d) means for calculating the pharmacokinetic property of a trial molecule using the above obtained QSPR model.

Another aspect of this invention provides a method wherein the pharmacokinetic property is absorption.

Another aspect of this invention provides a method wherein the pharmacokinetic property is distribution.

Another aspect of this invention provides a method wherein the pharmacokinetic property is metabolism

Another aspect of this invention provides a method wherein the pharmacokinetic property is excretion.

Another aspect of this invention provides a method wherein the internally developed macro comprises the macro script 2dfp.spl or 2dfp_abs.spl, written in SYBYL™ Programming Language (SPL).

Preferably, each of the steps of the methods of the invention is carried out using molecular modeling software, databases or drawing software. More preferably one is such as SYBYL™, version 6.5 (Tripos Inc., St. Louis, MO). The database includes, for example, ISIS Bases version 2.2.1 (MDL information Systems, Inc. San Leandro, CA.). The drawing software includes such as SYBYL™/SKETCH option, ISIS™ Draw version 2.2.1, Chem Draw Pro™ version 5.0 (CambridgeSoft. Corp. Cambridge, MA) and SMILES™ (Daylight Chemical Information Systems, Inc., Mission Viejo, CA). Other modeling software, databases, and drawing software known to those of skill in the art can also be used.

This invention enables us to perform virtual screening for synthetic targets and data mining using databases as well as drug design to optimize the pharmacokinetic profiles. Based on the QSPR model in this invention, it is possible to predict pharamacokinetic properties of molecules prior to synthesis, without labor-intensive and time-consuming experiment. This invention relies on 2D-fingerprint modeling requiring only 2D-structure, which enables us to perform rapid calculation to predict hundreds of compounds without tedious calculation about 3D-structure. Moreover, 2D-fingerprint used in this invention comprises only 20-80 bits.

### Description of Figures

Figure 1 is a flowchart showing the overall process of the invention.
Figure 2 shows a plot of actual vs. calculated log t1/2.
Figure 3 shows a plot of actual vs. calculated log(Pₐₚₚ * 10⁶).

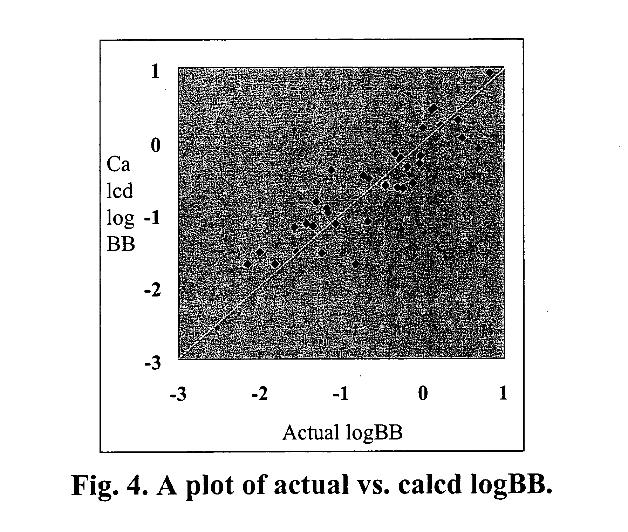

Figure 4 shows a plot of actual vs. calculated logBB.

### Detailed Disclosure of the Invention

The term "molecules used as a training set" as used herein, refers to the molecules whose pharmacokinetic properties have been already determined experimentally and used to develop a predictive QSPR model.

The term "pharmacokinetic properties" as used herein, refers to the properties of molecules related to metabolism, absorption (permeability), distribution, and excretion (ADME).

A number of experimental methods or models are known in ADME.

Examples of absorption studies are 1) kinetic studies based on measuring plasma concentration, urinary fecal excretions and gastrointestinal disposition after oral administration *in vivo,* 2) single-pass perfusion method, recirculation method, loop method *in situ*, and 3) everted sacs method, methods of using brush border membrane vesicles, isolated cells, and cultured cells (Caco-2) *in vitro* and the like.

Examples of distribution studies are 1) the method of measuring concentration of target organs after administration by various technique such as HPLC, LC-MS, autoradiography and microdialysis *in vivo,* 2) brain perfusion methods such as vascular reference method (brain uptake index) *in situ*, and 3) methods of using isolated cells or cultured cells (such as endothelial cell) *in vitro* and the like.

Examples of metabolism studies are 1) kinetic studies based on measuring concentrations of drugs and the metabolites after adequate administration routes such as intravenous administration, administration per portal vain *in vivo,* and *in situ*, 2) kinetic studies such as a half-life of drugs in mammalian organ (liver, kidney, intestine, etc. with slices, homogenates and microsomes etc) and in isolated cells or cultured cells such as hepatocytes *in vitro* and the like.

Examples of excretion studies are 1) kinetic studies based on measuring concentration of drugs in urine, bile, feces etc after administration *in vivo,* 2) enzymatic studies of excretion via pumps such as P-glycoprotein, *in vitro* and the like.

The term "2D-fingerprint" as used herein, refers to a 2D-molecular measure in which a bit in a data string is set corresponding to atoms/fragments or substructures.

The term "predefined atoms/fragments or substructures" as used herein, refers to atoms or functional groups relating to a phrmacokinetic property, which are based on the literature source (Bonse, V.G., Metzler, M. "Biotransformationen Organischer Fremdsubstanzen" (Yakubutu-Taisha) in Japanese Asakura, Tokyo (**1980**); Kato, R., Kamatani, T. "Yakubutu-Taishagaku" in Japanese, Tokyo-Kagaku-Dojin, Tokyo, chapter 4, 93-123 ,(**1995**)), otherwise refers to functional groups such as saturated or unsaturated bonds, rings (aromatic or cycloalkyl), amines, anilines, nittrogen in aromatics, imines/nitriles/guanidine/amidine, oxyamine(N-O)/nitro/azo-/hydrazin, amide/thioamide/sulfonamide/, alcohol/ether/aldehyde/ketone/ester/carboxylic acid/carbothioic acid/sulfinic acid/sulfonic acid, halogen, oxygen or sulfur functional groups, and total number of carbon, hydrogen, nitrogen, oxygen, sulfur or phosphorus atom.

The term "internally developed macro" as used herein, refers to an internally developed Sybyl Programming Language (SPL) code. Preferable internally developed macro is as described in Working Examples 4 and 5.

The QSPR model based on 2D-fingerprints for metabolism predicts half-life of molecules in a human liver microsome mixture with good predictivity. The 2D-fingerprints for absorption are successfully employed to develop a higly predictive QSPR model on drug permeability across monolayer Caco-2 cells. Similarly the present 2D-fingerprints/PLS modeling can be applied to develop statistically significant QSPR models on blood-brain barrier partitioning of the structurally diverse set. Thus, the method of this invention requiring only 2D-structures of the pertinent molecules enables to perform virtual screening of synthetic targets and data mining using molecular database as well as drug design to optimize the pharmacokinetic profiles.

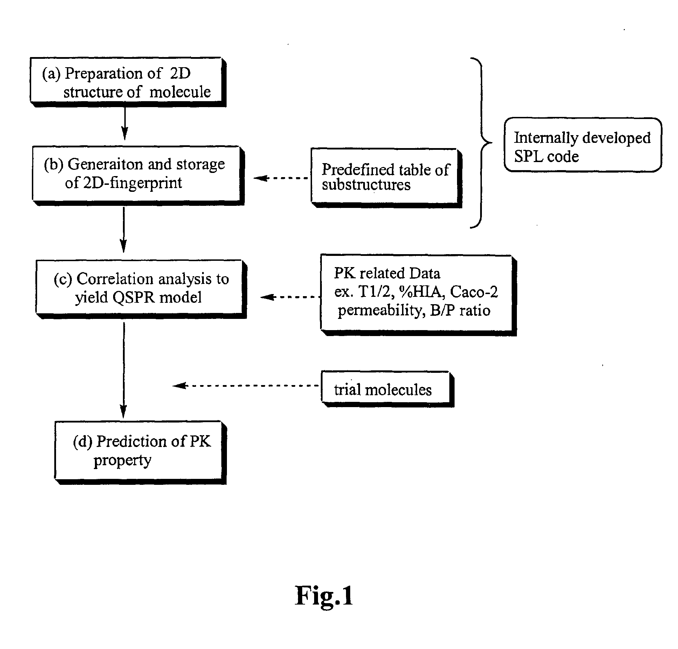

Figure 1 illustrates the method of this invention. This invention will be described in more detail with reference to Figure 1. Computational modeling studies can be carried out using molecular modeling software, preferably SYBYL™ on a Silicon Graphics Octane™ workstation. The method of this invention comprises the following steps:
(a) 2D-structure of a molecule can be prepared by retrieving from a database such as ISIS™/Base, or by constructing manually with drawing software. The drawing software includes, for example, SYBYL™/SKETCH option (on the workstation), or ISIS™ Draw, Chem Draw™ and SMILES™ on (PC such as Windows NT client PC). The 2D-structure thus prepared can be transferred to the workstation, and stored in the molecular database.
(b) The prepared 2D-structure of a molecule can be imported into molecular modeling software such as SYBYL™ as a MOL2 format. 2D-fingerprints can be constructed by the use of internally developed macro script 2dfp.spl or 2dfp_abs.spl, written in SYBYL™ Programming Language (SPL) implemented in SYBYL™, or by manually counting the number of the atoms/fragments or substructures. The macro program converts 2D-structures stored in the molecular database as a MOL2 format into a SYBYL™ line notation (SLN) format. Subsequently, the macro searches each SLN for the substructures potentially related to a pharmacokinetic property that match the queries described in the macro (as shown in Working Example 4), wherein the queries are predefined as the substructures (20 to 80 atoms/fragments). Finally the macro enumerates the substructure counts, and records them as 2D-fingerprints.
(c) Statistical analysis is performed to obtain a correlation between the obtained 2D-fingerprints and the pharmacokinetic property. Any analytical method such as partial least square (PLS) algorithm, sample-distance partial least squares (SAMPLS; Bush, B. L. *et al. J. Computer-Aided Mol. Design,* **1993,** 7, 587-619), genetic algorithm or neural network can be employed to yield an optimal quantitative structure property relationship (QSPR) model.
(d) The pharmacokinetic property for trial molecules can be calculated based on the above obtained QSPR model.

The pharmacokinetic properties of the molecule such as absorption, distribution, metabolism and excretion, can be apparent permeability coefficients (Pₐₚₚ) [cm/sec], blood-brain barrier partitioning ratio {(C_{brain}/C_{blood}) = BB}, half-life(T_{1/2}) in mammalian liver microsome and the like.

The system of this invention can be constructed using appropriate computer hardware such as a Silicon Graphics Octane™ workstation and software as described above.

This invention will be further described below with reference to the following Working Examples.

### Examples

### Example 1

### Development and validation of QSPR for half life in human liver microsome.

Computational modeling studies were carried out using a Silicon Graphics Octane™ workstation. A congeneric series of 54 compounds of Formula (I)(as shown in the following Table 1.) with a variety of substituent groups were used as a training set for analysis.

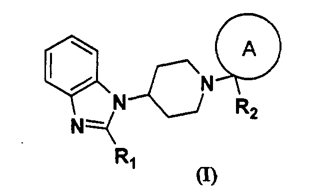

**Table 1.**

| # | A | R1 | R2 |
|---|---|---|---|
| 1a | cycloheptyl | piperidinyl | Ph |
| 2a | cycloheptyl | H₂N(CH₂)₂O- | Ph |
| 3a | cycloheptyl | 4-aminopiperidyl | Ph |
| 4a | cycloheptyl | H₂N(CH₂)₂C(O)- | Ph |
| 5a | cycloheptyl | H2N(CH2)2CONH- | Ph |
| 6a | cyclohepten-1-yl | 4-aminopiperidyl | Ph |
| 7a | cyclooctyl | H₂NCH₂CONH- | Ph |
| 8a | cycloheptyl | H₂N(CH₂)₃- | Ph |
| 9a | cycloheptyl | 4-aminocyclohexylamino | Ph |
| 10a | cyclohepten-1-yl | piperazinyl | Ph |
| 11 a | cycloheptyl | piperazinyl | Ph |
| 12a | cycloheptyl | H₂N(CH₂)₂NH- | Ph |

| | | | |
|---|---|---|---|
| 13a | cycloheptyl | H2NC(CH3)2CH2NH- | Ph |
| 14a | cycloheptyl | N-methylpiperazinyl | Ph |
| 15a | cycloheptyl | piperidinylamino | Ph |
| 16a | cycloheptyl | 4-aminopiperidyl | CH₃ |
| 17a | cycloheptyl | piperidinyl | CH₃ |
| 18a | cycloheptyl | H₂N(CH₂)₁₀NH- | Ph |
| 19a | cycloheptyl | 4-aminoazetidinyl | Ph |
| 20a | cycloheptyl | H₂N(CH₂)₈NH- | Ph |
| 21a | cycloheptyl | (CH₃)₂N(CH₂)₂NH- | Ph |
| 22a | cyclooctyl | N-methylpiperazinyl | Ph |
| 23a | cycloheptyl | piperazinyl | isopropyl |
| 24a | cycloheptyl | piperidinecarboximidamide | Ph |
| 25a | cycloheptyl | H2N(CH2)6NH- | Ph |
| 26a | cycloheptyl | H2N(CH2)4NH- | Ph |
| 27a | cyclononyl | amino | Ph |
| 28a | cycloheptyl | CH₃NH(CH₂)₂NH- | Ph |
| 29a | cyclooctyl | piperazinyl | CH₃ |
| 30a | cycloheptyl | 4-aminopiperidyl | vinyl |
| 31a | cycloheptyl | isopropyl | Ph |
| 32a | cycloheptyl | 2-guanidinoethyl | Ph |
| 33a | cycloheptyl | mathanesulfoonyl | Ph |
| 34a | cycloheptyl | piperidinyloxy | Ph |
| 35a | cycloheptyl | dimethylamino | Ph |
| 36a | cycloheptyl | amino | Ph |
| 37a | cycloheptyl | CH₃CONH- | Ph |
| 38a | cycloheptyl | hydroxypiperidinyl | Ph |
| 39a | cycloheptyl | H₂N(CH₂)₃SO₂- | Ph |
| 40a | cycloheptyl | methylamino | Ph |
| 41a | cycloheptyl | methyl | Ph |
| 42a | cyclooctyl | piperazinyl | CH₃ |
| 43a | cycloheptyl | isobutyl(NH₂)CHCONH- | Ph |
| 44a | cycloheptyl | methylamino | CH₃ |
| 45a | cycloheptyl | methoxy | Ph |
| 46a | cyclooctyl | methylamino | normalpropyl |

| | | | |
|---|---|---|---|
| 47a | cyclooctyl | methylamino | CH₃ |
| 48a | cyclooctyl | methylpiperazinyl | CH₃ |
| 49a | cycloheptyl | H | Ph |
| 50a | cyclononyl | methylamino | CH₃ |
| 51a | cyclononyl | methylpiperazinyl | CH₃ |
| 52a | cycloheptyl | isobutyl(NH₂)CHCONH- | CH₃ |
| 53a | cycloheptyl | H2N(CH2)2CONH- | Ph |
| 54a | cycloheptyl | H₂N(CH₃)₂CCONH- | Ph |

Half-life (t1/2) *in vitro* for each compound was determined by HPLC analysis of the reaction mixture with human liver microsome. The employed 2D-structures were retrieved from ISIS™/Base (version 2.2.1, MDL Information Systems, Inc., San Leandro, CA) or constructed with ISIS™/Draw (version 2.2.1, MDL Information Systems, Inc., San Leandro, CA) on a WinNT client PC, followed by being transferred to the Octane workstation and stored in a molecular database. The 2D-fingerprints were constructed by use of a newly developed macro script 2dfp.spl, written in SYBYL™ Programming Language (SPL), which was implemented in SYBYL™ (version 6.5, Tripos Inc., St. Louis, MO). The macro program converted 2D-structures stored in the molecular database as MOL or MOL2 format into SYBYL™ line notation (SLN) format, and counted the number of the atoms or functional groups that matched queries defined in a table described in the macro program. The atoms or functional groups susceptible to be involved in metabolism were assigned on the basis of the literature source (Bonse, V. G., Metzler, M. "Biotransformationen Organischer Fremdsubstanzen" (Yakubutu-Taisya, in Japanese) Asakura, Tokyo (1980); Kato, R.; Kamataki, T. "Yakubutu-Taisyagaku" in Japanese, Tokyo-Kagaku-Dojin Tokyo (1995)). Partial least square (PLS) algorithm in QSPR module in SYBYL™ was employed to correlate the aforementioned 2D-fingerprints and t1/2 to produce QSPR model. Thirty-eight bits out of whole 2D-fingerprints used since 25 bits with all the same value or 0 were dropped. SAMPLS run in crossvalidation step (leave-1-out) identified the optimum PLS component as 5 (N = 54, Std. Error_prediction = 0.414; q² = 0.518). Non-crossvalidation PLS analysis resulted in a significant five-component model with the following statistics: Std. Error_Est. = 0.219, r² = 0.865, F(nl = 5, n2 = 48) = 61.3.

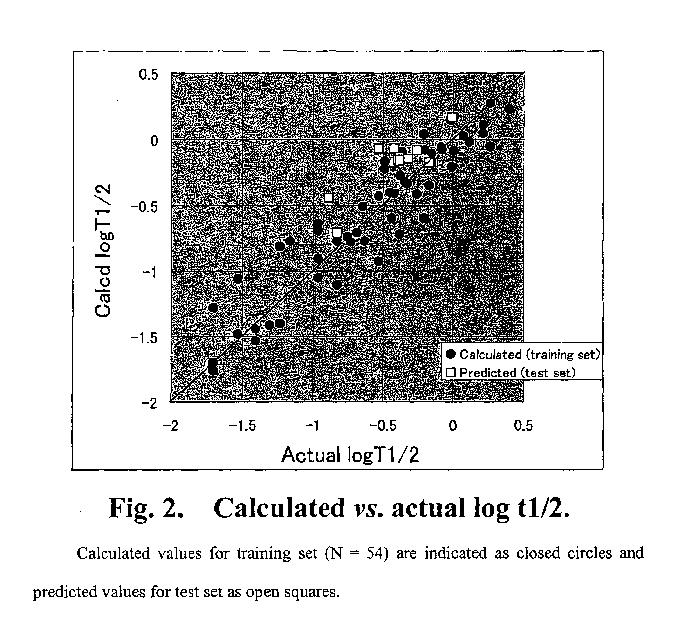

Figure 2 shows the plot of actual vs. calculated log t1/2 (closed circles). For validation of the present QSPR model, the prediction of half-life for the test set (12 compounds) was performed. As indicated open squares in Figure 2, the model has a fairly good predictivity, which allows us to prioritize the targets for synthesis.

### Example 2

### Development of QSPR for Caco-2 permeability.

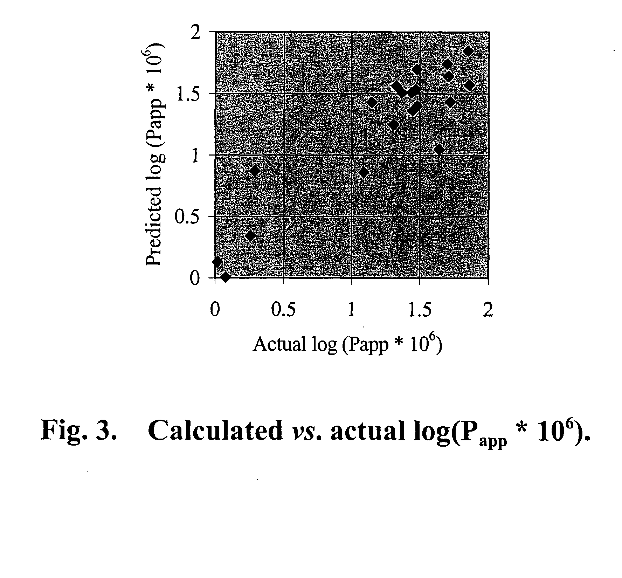

Unless otherwise noted similar computational molecular modeling were performed as described in Example 1. Table 2 enlists 21 structurally diverse compounds as a training set, whose apparent permeability coefficients (Pₐₚₚ) [cm/sec] of a compound across Caco-2 cells was used as in literature source (Yee, S. *Pharm. Res.* **1997,** 14, 763―766). The counts of substructures to match with the predefined queries were encoded as a array of integers by a similar SPL script (2dfp_abs.spl) to afford 2D-fingerprints as descriptors employed in the correlation analysis. SAMPLS run in crossvalidation step (leave-1-out) identified the optimum PLS component as 2 (N = 21, Std. Error_prediction = 0.444; q² = 0.463). Non-crossvalidation PLS analysis resulted in a significant two-component model with the following statistics: Std. Error_Est. = 0.254, r² = 0.824, F(nl = 2, n2 = 18) = 42.1. Figure 3 shows the plot of actual vs. calculated log(Pₐₚₚ * 10⁶).

**Table 2.**

| **Training set compounds with apparent permeability.** | | | | | |
|---|---|---|---|---|---|
| | Pₐₚₚ * 10⁶ | | Pₐₚₚ * 10⁶ | | Pₐₚₚ * 10⁶ |
| Compd. | | Compd. | | Compd. | |
| | (cm/sec) | | (cm/sec) | | (cm/sec) |
| Azithromycin | 1.04 | Diazepam | 70.97 | Prazosin | 43.60 |
| Benzylpenicillins | 1.96 | Erythromycin | 1.80 | Propranolol | 27.50 |
| Caffeine | 50.50 | Fluconazole | 29.80 | Quinidine | 20.40 |
| Chloramphenicol | 20.60 | Ibuprofen | 52.50 | Tenidap | 51.20 |
| Clonidine | 30.10 | Imipramine | 14.10 | Testosterone | 72.27 |
| Desipramine | 21.60 | Methotrexate | 1.20 | Trovafloxacin | 30.23 |
| Dexamethasone | 23.40 | Naloxone | 28.20 | Ziprasidone | 12.30 |

### Example 3

### Development of QSPR for blood-brain barrier partition.

Unless otherwise noted, similar molecular modeling was performed as described in Example 1. Blood-brain barrier partitioning ratio, {log(C_{brain}/C_{blood}) = logBB} for "drug-like" compounds (N = 35, Chart 1) as a training set were used as in literature source (Lombardo, F. *et al., J. Med. Chem.* **1996,** *39*, 4750―4755.). The 2D-fingerprints were calculated as above example. PLS modeling to correlate 2D-fingerprints with BBB partitioning ratio showed the following statistics. Crossvalidation (SAMPLS, leave-1-out): the optimum PLS component = 3, N = 35, Std. Error_prediction = 0.69; q² = 0.29. Non-crossvalidation: Std. Error_Est. = 0.38, r² = 0.78, F_{(3, 31)} = 37. 4.

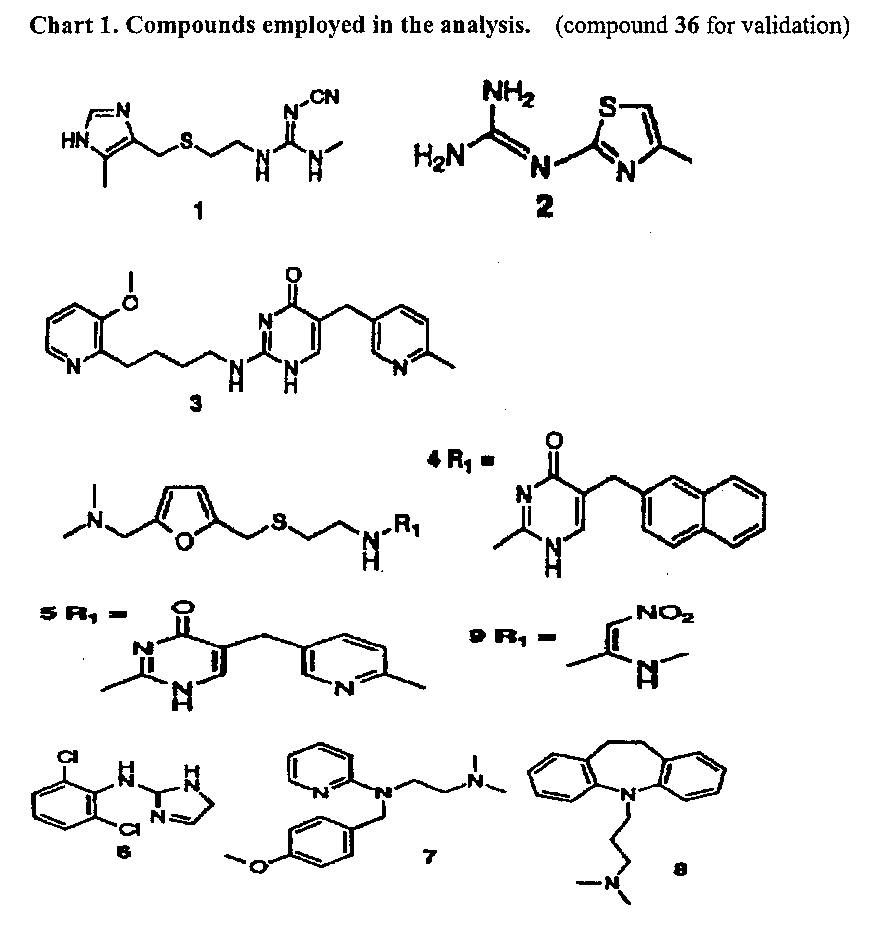

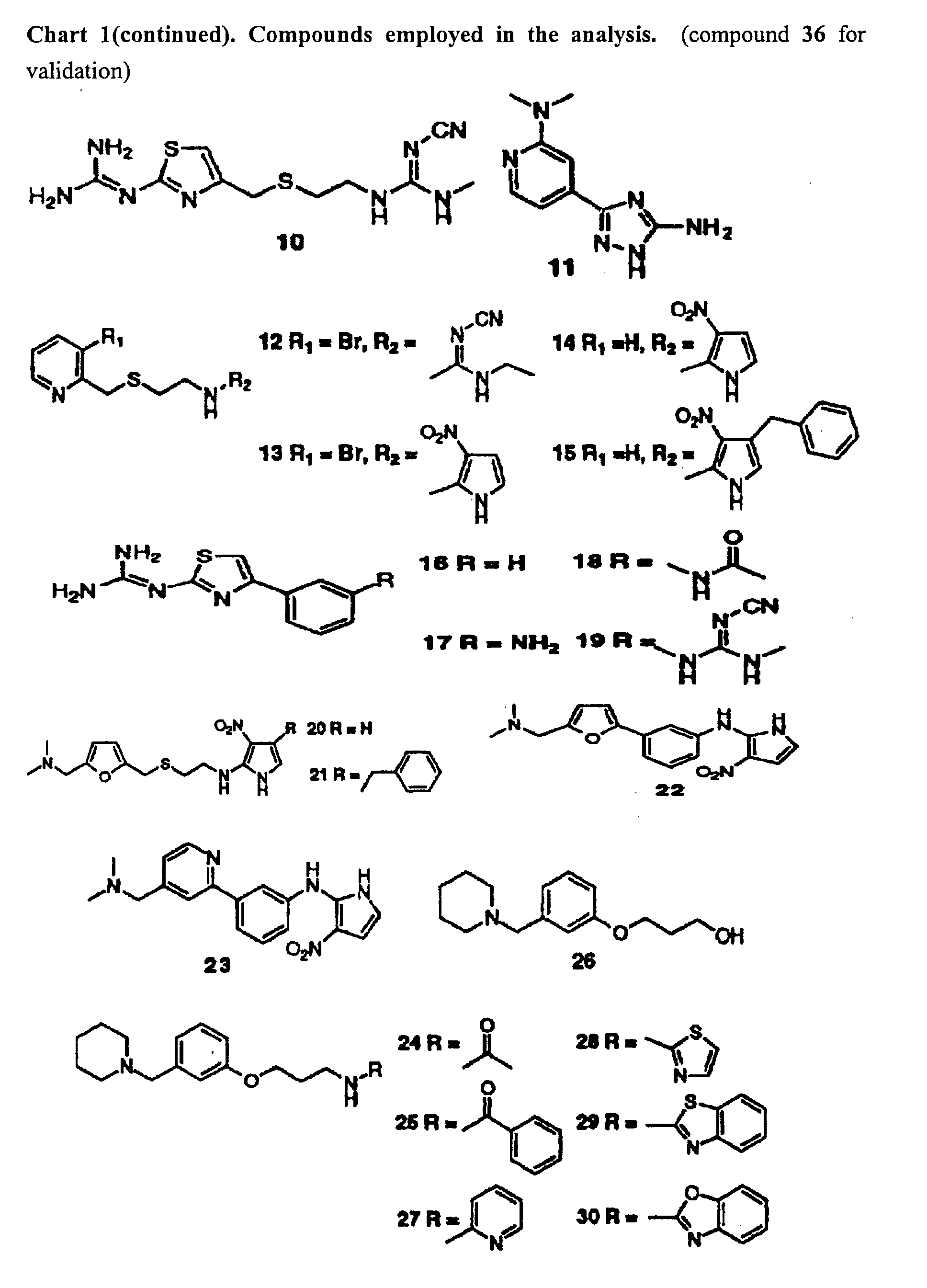

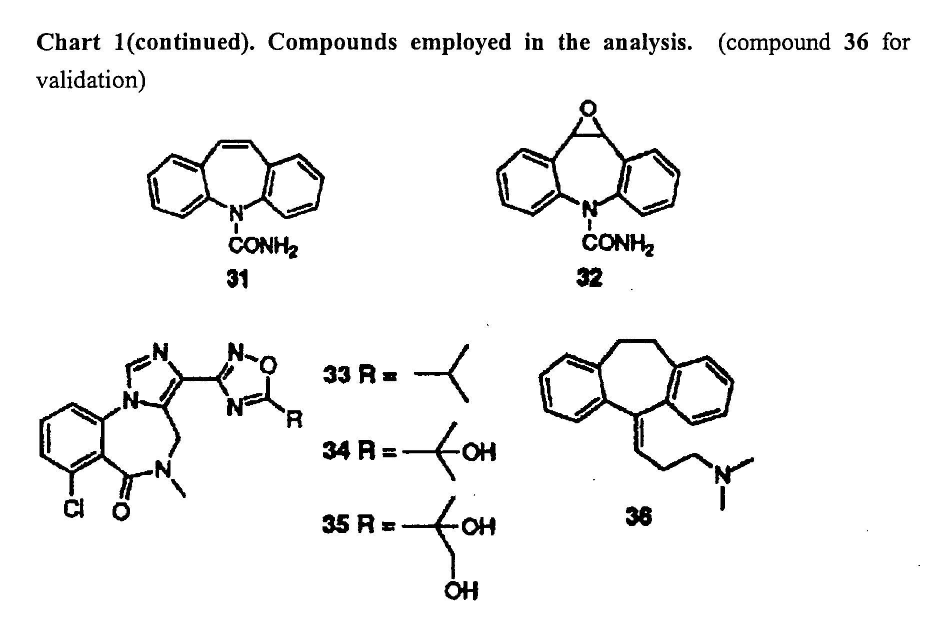

### Example 4

### SPL macro (2dfp.spl) to prepare 2D-fingerprints for half life.

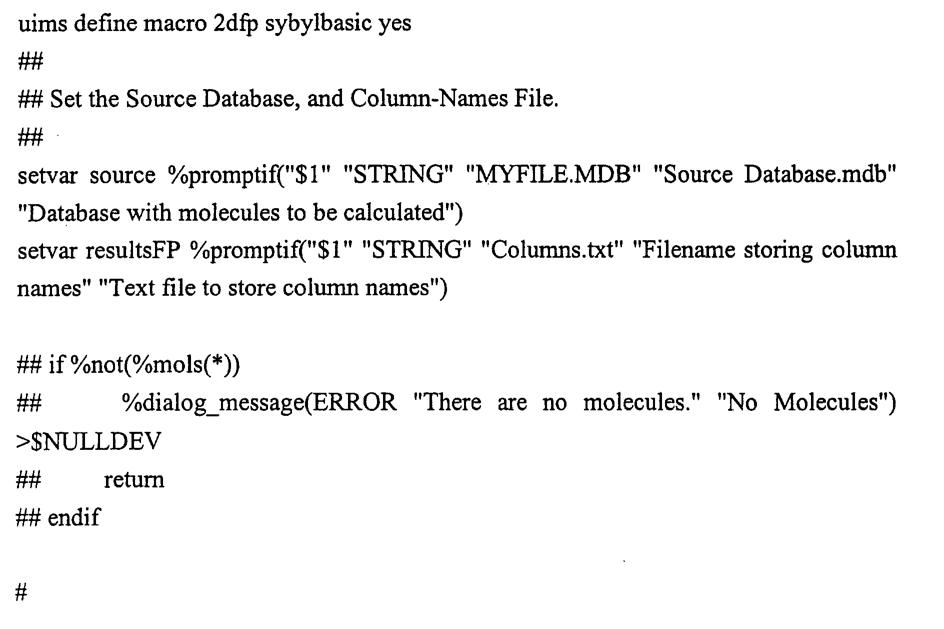

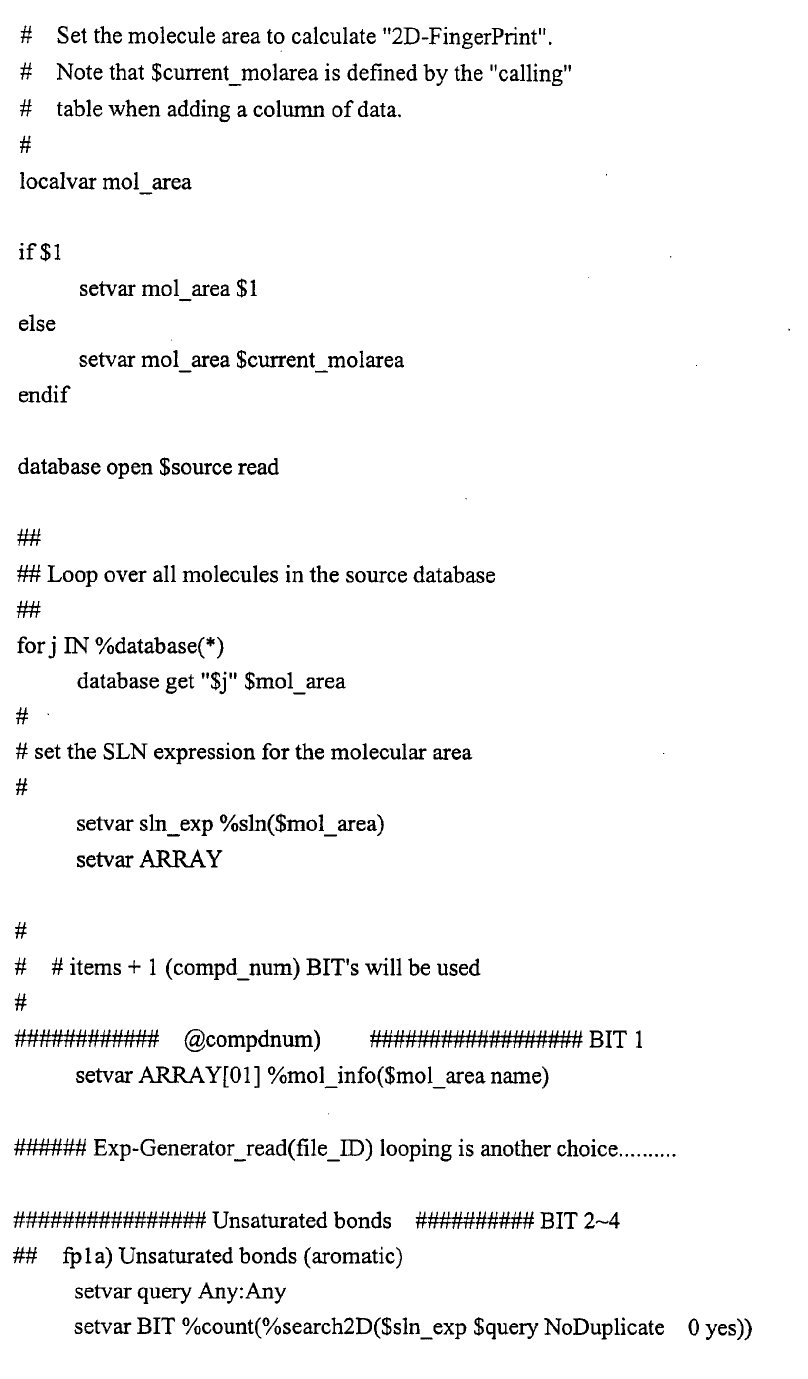

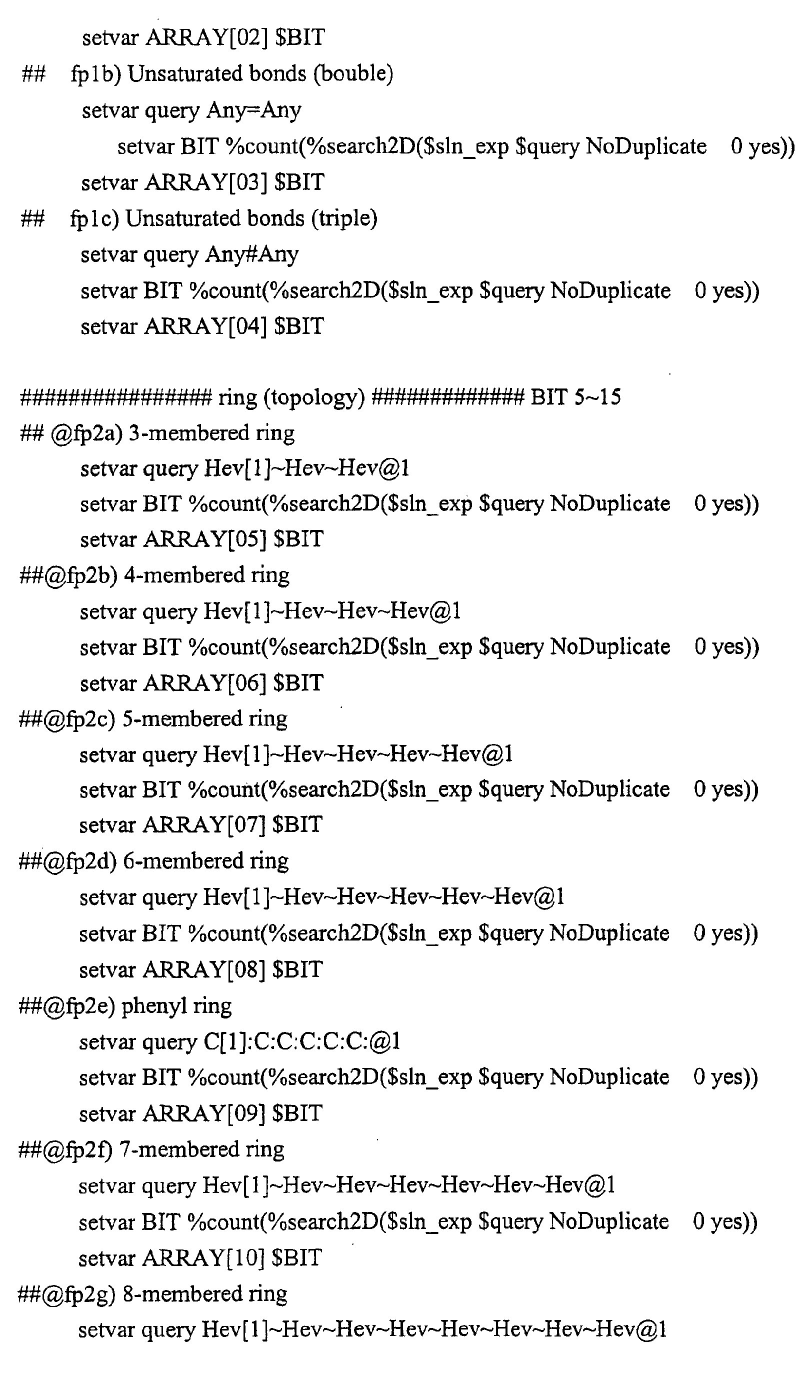

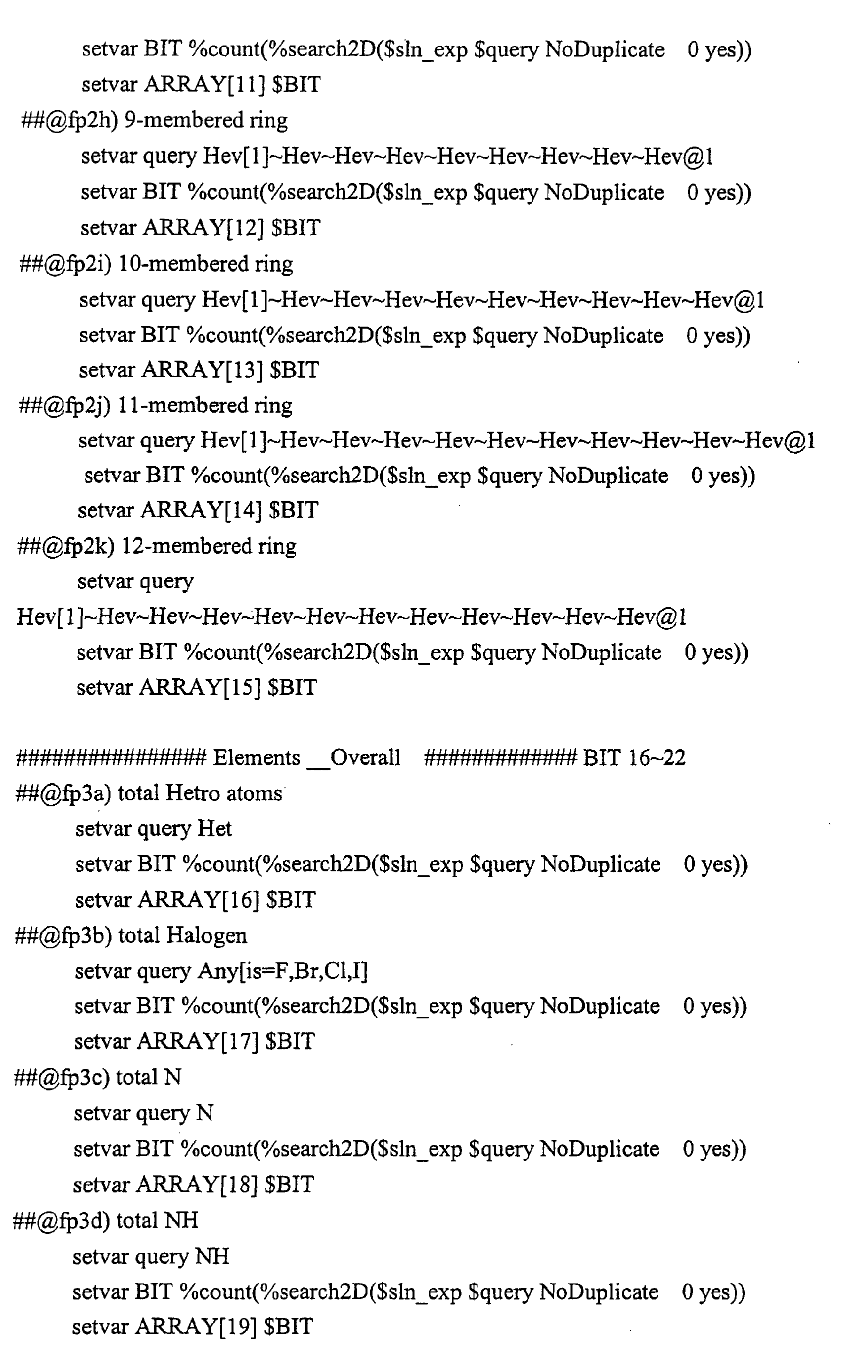

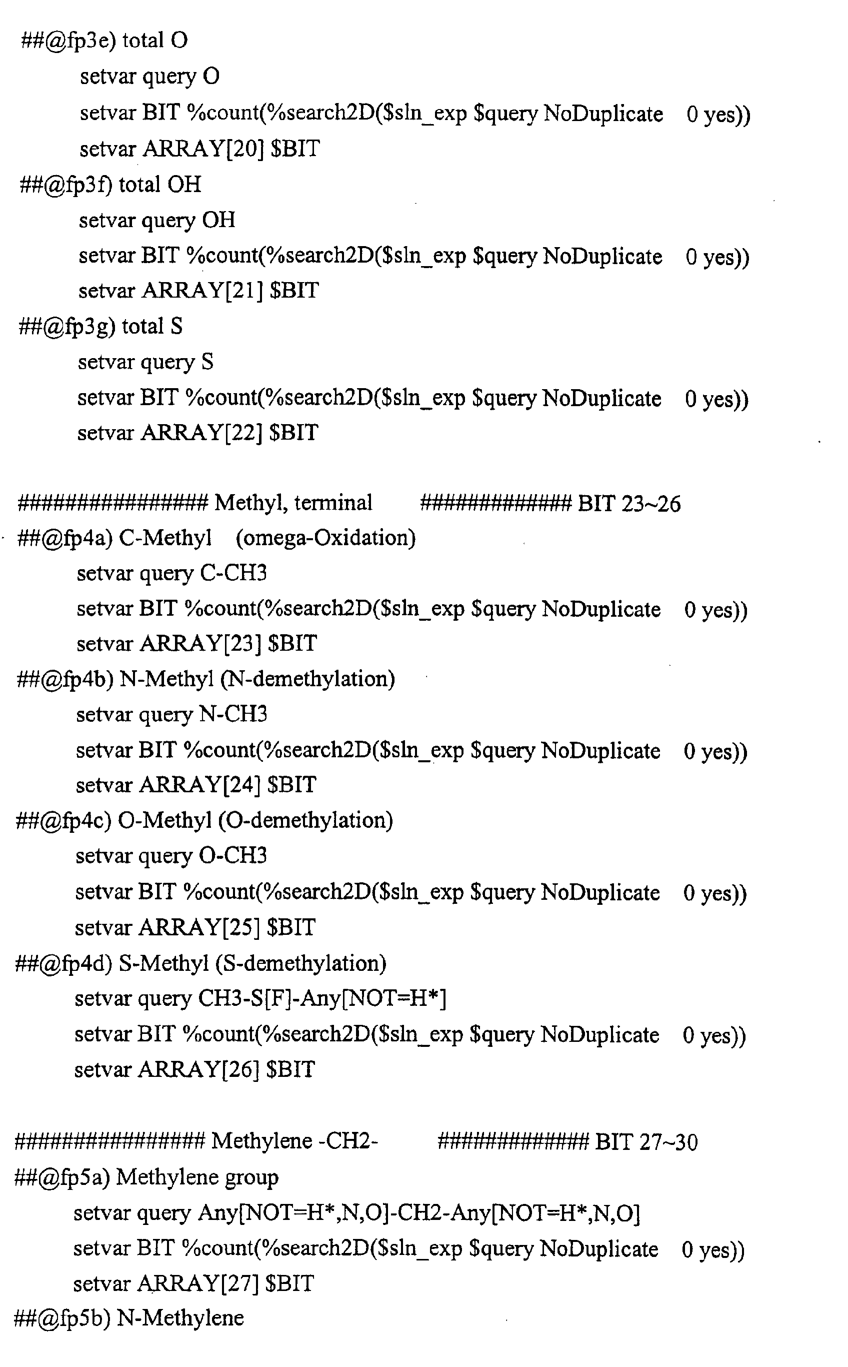

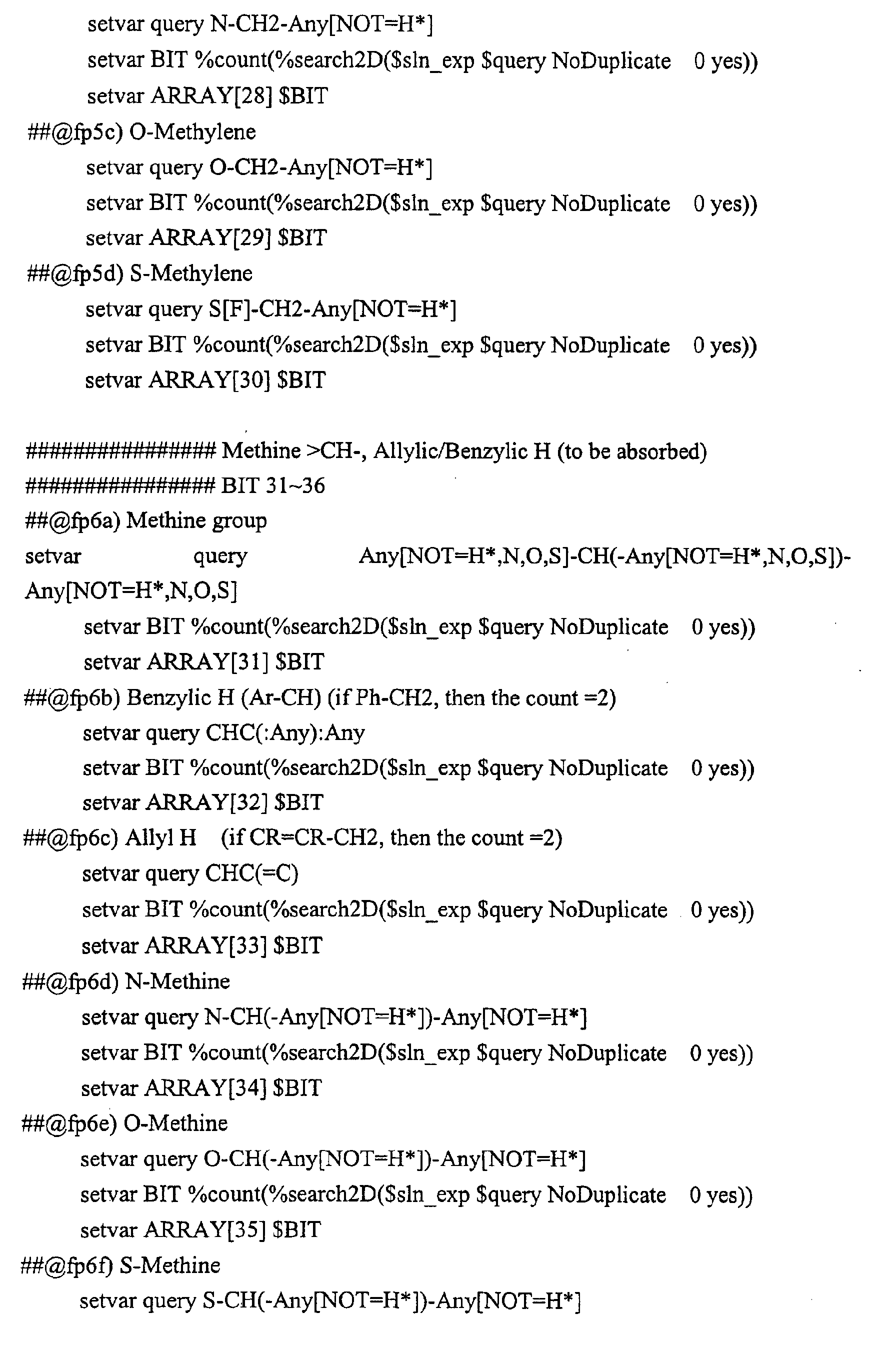

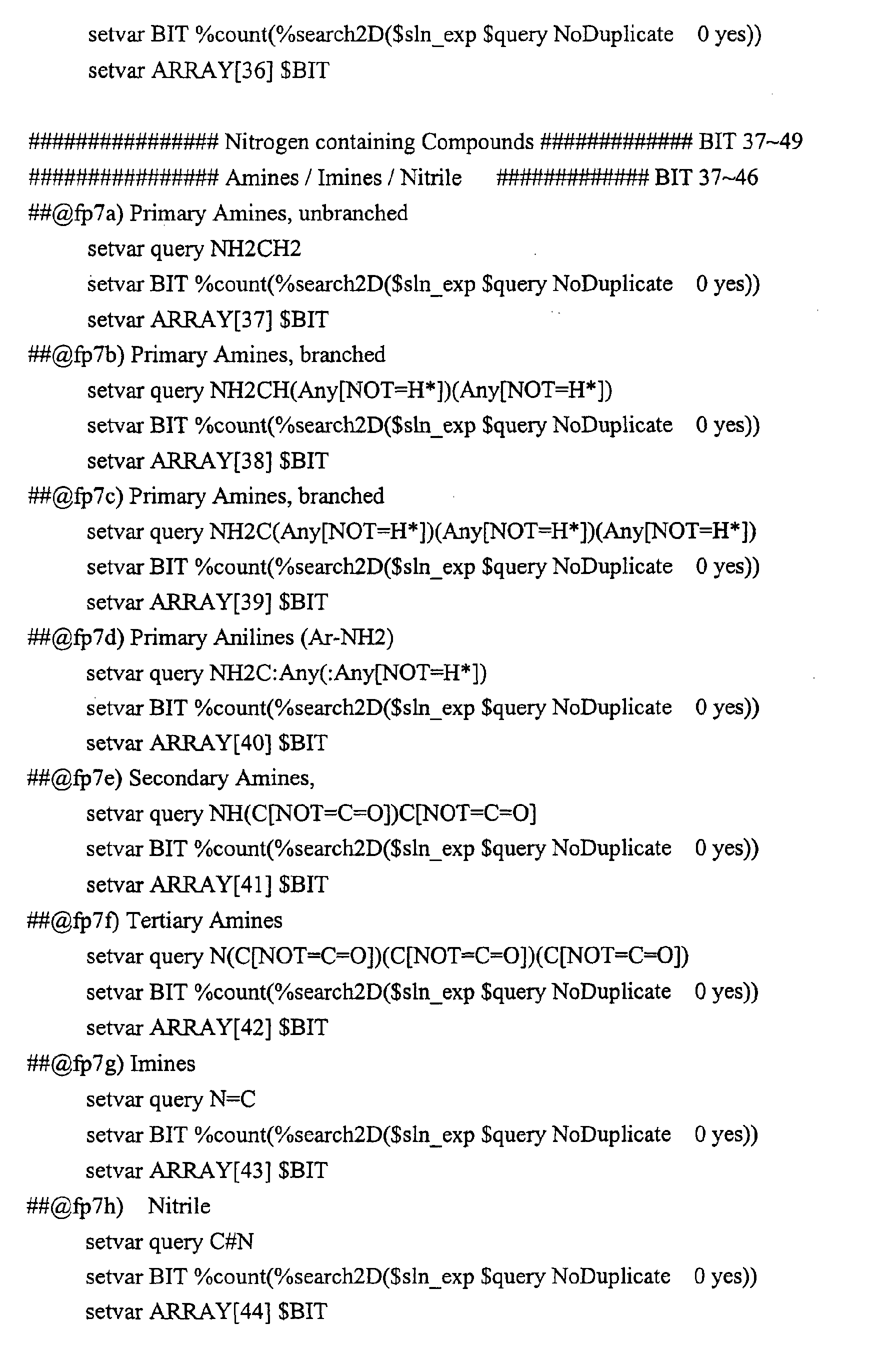

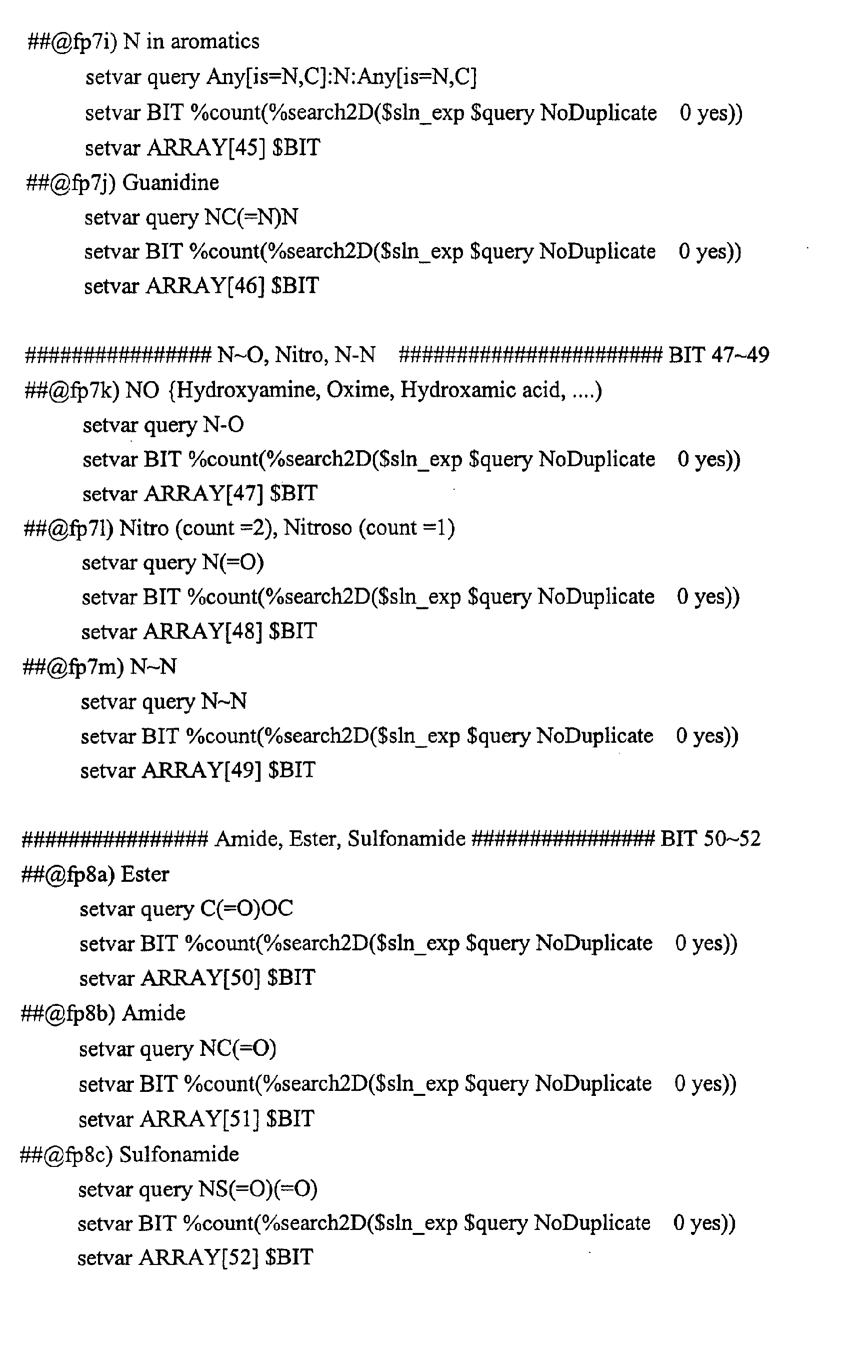

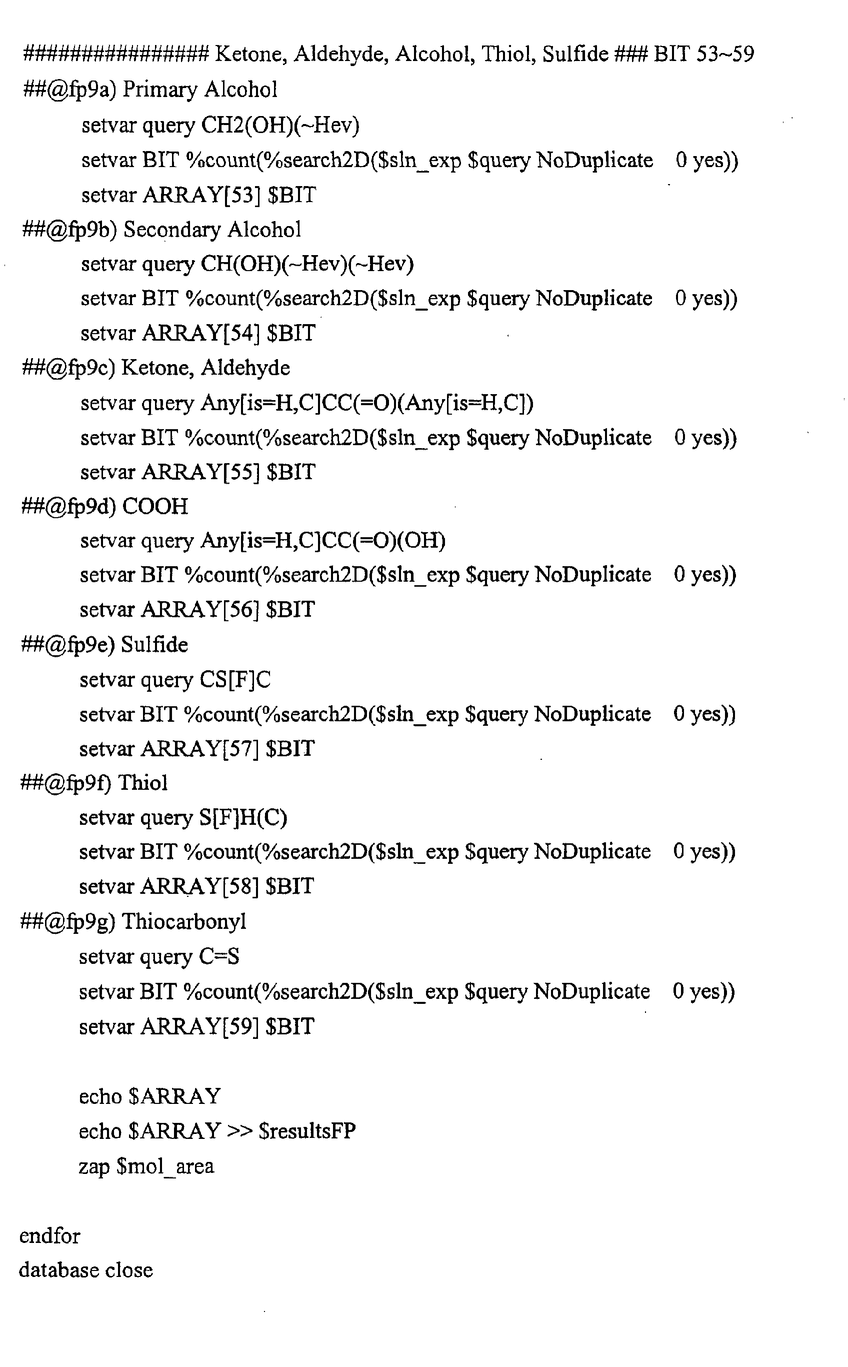

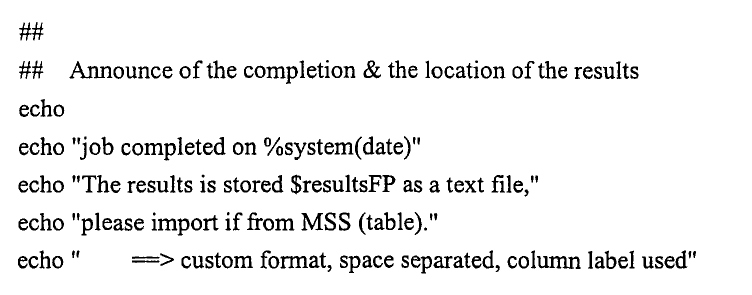

### Example 5

By use of a similar method described in example 4, 2D-Fingerprints for Caco-2 permeability and blood-brain barrier partition were prepared based on the following description.

| **Fp-ID** | **Name** | **Query** |
|---|---|---|
| *Alkyl Amines* | | |
| fp1a | Primary | NH2C[NOT=C=O,C=S,C:Any,Any[IS=C,N]C(=N)N,C[1](Any[IS=O,S,N]A ny=:AnyAny=@1),C[1](=AnyAny[IS=O,S,N]Any=:Any@1)] |
| fp1b | Secondary | NH(C[NOT=C=O,C=S,C:Any,Any[IS=C,N]C(=N)N,C[1](Any[IS=O,S,N]A ny=:AnyAny=@1),C[1](=AnyAny[IS=O,S,N]Any=:Any@1)])(C[NOT=C= O,C=S,C:Any,Any[IS=C,N]C(=N)N,C[1](Any[IS=O,S,N]Any=:AnyAny=@ 1),C[1](=AnyAny[IS=O,S,N]Any=:Any@1)]) |
| fp1c | Tertiary | N(C[NOT=C=O,C=S,C:Any,Any[IS=C,N]C(=N)N,C[1](Any[IS=O,S,N]An y=:AnyAny=@ 1),C[1](=AnyAny[IS=O,S,N]Any=:Any@1)])(C[NOT=C=O, C=S,C:Any,Any[IS=C,N]C(=N)N,C[1](Any[IS=O,S,N]Any=:AnyAny=@1) ,C[ 1](=AnyAny[IS=O,S,N]Any=:Any@1)])(C[NOT=C=O,C=S,C:Any,Any[ IS=C,N]C(=N)N,C[1](Any[IS=O,S,N]Any=:AnyAny=@1),C[1](=AnyAny[1 S=O,S,N]Any=:Any@1)]) |

| Amines attached to heteroaromatics | | |
|---|---|---|
| fp2a | Primary | C[1](Any[IS=O,S,N]Any=:AnyAny=@1)NH2 |
| | | C[ 1](=AnyAny[IS=O,S,N]Any=:Any@1)NH2 |
| fp2b | Secondary | C[1](Any[IS=O,S,N]Any=:AnyAny=@1)NHAny[NOT=H*] |
| | | C[1](=AnyAny[IS=O,S,N]Any=:Any@1)NHAny[NOT=H*] |
| fp2c | Tertiary | C[1](Any[IS=O,S,N]Any=:AnyAny=@1)N(Any[NOT=H*])Any[NOT=H*] |
| | | C[1](=AnyAny[IS=O,S,N]Any=:Any@1)N(Any[NOT=H*])Any[NOT=H*] |

| Anilines | | |
|---|---|---|
| fp3a | Primary | NH2C(:Any)(:Any[NOT=H*]) |
| fp3b | Secondary | NH(C(:Any)(:Any[NOT=H*]))Any[NOT=H*] |
| fp3c | Tertiary | N(C(:Any)(:Any[NOT=H*]))(Any[NOT=H*])Any[NOT=H*] |
| | | N(C(:Any)(:Any[NOT=H*]))=C |

| N in aromatics | | |
|---|---|---|
| fp4a | 6-membered ring | Any[is=N,C]:N:Any[is=N,C] |
| fp4b | -NH- in heteroaromatics | N[1]HAny[IS=C,N]=:Any[IS=C,N]Any[IS=C,N]=:Any[IS=C,N]-@1 |
| fp4c | -N- in heteroaromatics | N[ I ]Any[IS=C,N]=:Any[IS=C,N]Any[IS=C,N]=:Any[IS=C,N]-@1 |
| fp4d | -N= in heteroaromatics | N[1](Any[IS=O,S,N]Any=:AnyAny=@1) |
| | | N[1](=AnyAny[IS=O,S,N]Any=:Any@1) |

| ***Imines/Nitrile/ Guanidine/Amidine*** | | |
|---|---|---|
| fp5a | Imines | Any[IS=C,H,S]N[NOT=N[1](Any[IS=O,S,N]Any=:AnyAny=@1),N[1](=A nyAny[IS=O,S,N]Any=:Any@1)]=C[NOT=Any[IS=C,N]C(=N)N] |
| fp5b | Nitrile | C#N |
| fp5c | Guanidine | N[NOT=C[1](Any[IS=O,S,N]Any=:AnyAny=@1)N,C[1](=AnyAny[IS=O,S ,N]Any=:Any@1)N]C(=N)N[NOT=C[1](Any[IS=O,S,N]Any=:AnyAny=@ 1)N,C[1](=AnyAny[IS=O,S,N]Any=:Any@1)N] |
| fp5d | Amidine (not hetero-aromatics) | Any[NOT=N]C(=N[NOT=N[1](Any[IS=O,S,N]Any=:AnyAny=@1),N[1](= AnyAny[IS=O,S,N]Any=:Any@1)])N |

| ***N∼O/Nitro/N=N/N-N*** | | |
|---|---|---|
| fp6a | Hydroxyamine, Oxime, Hydroxamic acid....) | N[!r]-O[!r] |
| fp6b | Nitro, Nitroso | N(=O) |
| fp6c | N=N Azo (not in a ring) | N=N[NOT=N[1](Any[IS=O,S,N]Any=:AnyAny=@1),N[1](=AnyAny[IS=O ,S,N]Any=:Any@1)] |
| fp6d | N-N Hydrazine | N-N[NOT=N[1](Any[IS=O,S,N]Any=:AnyAny=@1),N[1](=AnyAny [IS=O,S,N]Any=:Any@1)] |

| **Amide/ Thioamide/ Sulfonamide** | | |
|---|---|---|
| fp7a | Amide1 (NH₂-CO) | NH2C=O |
| fp7b | Amide2 (R₁-NH-CO) | Any[NOT=H*]NHC=O |
| fp7c | Amide3 (R₁R₂N-CO) | Any[NOT=H*]N(C=O) Any[NOT=H*] |
| fp7d | Thioamidel (NH₂-CS) | NH2C=S |
| fp7e | Thioamide2 (R₁-NH-CS) | Any[NOT=H*]NHC=S |
| fp7f | Thioamide3(R₁R₂N-CS) | Any(NOT=H*]N(C=S)Any(NOT=H*] |
| fp7g | Sulf.amidel (NH₂SO₂) | NH2S(=O)(=O) |
| fp7h | Sulf.amide2 (R₁-NHSO₂) | NH(S(=O)=O)Any[NOT=H*] |
| fp7i | Sulf.amide3 (R₁R₂-NSO₂) | N(S(=O)=O)(Any[NOT=H*])Any[NOT=H*] |

| ***Alcohol/Ether/Aldehyde/Ketone/Ester/Carboxylic acid/Carbothioic acid/Sulfinic acid/Sulfonic acid*** | | |
|---|---|---|
| fp8a | Alcohol | C[NOT=C=O,C=S](OH) |
| fp8b | Ether | Any[NOT=C=O, H*]-O-Any[NOT=C=O,H*] |
| fp8c | Aldehyde | CCH(=O) |
| fp8d | Ketone | CC(=O)C |
| fp8e | Ester | C(=O)OC |
| fp8f | Carboxylic acid | C(=O)(OH) |
| fp8g | Carbothioic O acid | C(=S)(OH) |
| fp8h | Carbothioic S acid | C(=O)(SH) |
| fp8i | sulfinic acid | Any[is=H,C]S[NOT=S(=O)(=O)](=O)(OH) |
| fp8j | sulfonic acid | Any[is=H,C]S(=O)(=O)(OH) |

| Halogen | | |
|---|---|---|
| fp9a | Fluoro | F |
| fp9b | Chloro | Cl |
| fp9c | Bromo | Br |
| fp9d | Iodo | I |

| ***Total C/H/N/O/S*** | | |
|---|---|---|
| fp10a | total C | C |
| fp10b | total H | H |
| fp10c | total N | N |
| fp10d | total O | O |
| fp10e | total S | S |

## Claims

1. A method for predicting pharmacokinetic properties of molecules comprising the steps of:
(a) preparing 2D-structures of molecules used as a training set;
(b) constructing a 2D-fingerprint by counting the number of structural descriptors that potentially relate to a pharmacokinetic property, either manually or automatically using internally developed macro; wherein said structural descriptors consist of predefined 20 to 80 atoms/fragments or substructures;
(c) analyzing the obtained 2D-fingerprint by a statistical analysis method to correlate with the pharmacokinetic property of the molecule to yield a quantitative structure-property relationship (QSPR) model; and
(d) calculating the pharmacokinetic property of a trial molecule using the above obtained QSPR model.

2. A method of Claim 1, wherein the pharmacokinetic property is absorption.

3. A method of Claim 1, wherein the pharmacokinetic property is distribution.

4. A method of Claim 1, wherein the pharmacokinetic property is metabolism

5. A method of Claim 1, wherein the pharmacokinetic property is excretion.

6. A method of Claim 1, wherein the internally developed macro comprises the macro script 2dfp.spl or 2dfp_abs.spl, written in SYBYL™ Programming Language (SPL).

7. A system for predicting pharmacokinetic properties of molecules comprising:
(a) means for preparing 2D-structures of molecules used as a training set;
(b) means for constructing a 2D-fingerprint by counting the number of structural descriptors that potentially relate to a pharmacokinetic property, wherein said structural descriptors consist of predefined 20 to 80 atoms/fragments or substructures;
(c) means for analyzing the obtained 2D-fingerprint by a statistical analysis method to correlate with the pharmacokinetic property of the molecule to yield a quantitative structure-property relationship (QSPR) model; and
(d) means for calculating the pharmacokinetic property of a trial molecule using the above obtained QSPR model.
